# EUROPEAN PATENT SPECIFICATION

(11) **EP 3 980 026 B1**
(45) Date of publication and mention of the grant of the patent: **31.07.2024**
(21) Application number: 20728800.2
(22) Date of filing: 03.06.2020
(51) Int. Cl.: A61K 31/706, A61P 13/12, A61P 35/00, A61P 43/00, A61K 9/00, A23L 2/00

(54) **REDUCED NICOTINAMIDE RIBOSIDES FOR TREATING OR PREVENTING KIDNEY DISEASE**
REDUZIERTE NICOTINAMIDERIBOSIDE ZUR BEHANDLUNG ODER PRÄVENTION VON NIERENERKRANKUNG
RIBOSIDES DE NICOTINAMIDE RÉDUITS DESTINÉES AU TRAITEMENT OU À LA PRÉVENTION DES MALADIES RÉNALES

(30) Priority: 05.06.2019 EP 19178423
(43) Date of publication of application: 13.04.2022
(73) Proprietor: Société des Produits Nestlé S.A., 1800 Vevey (CH)
(72) Inventor: CANTO ALVAREZ, Carles, 1148 Cuarnens (CH); CHRISTEN, Stefan, 1024 Ecublens (CH); GINER, Maria Pilar, 1005 Lausanne (CH); GIROUD-GERBETANT, Judith, 08700 Igualada Catalunya (ES); MOCO, Sofia, 1003 Lausanne (CH); BARTOVA, Simona, 1025 Saint-Suplice (CH); MIGAUD, Marie, Mobile, AL 36693 (US)
(74) Representative: Kamibayashi, Lynne
(86) International application number: PCT/EP2020/065332
(87) International publication number: WO 2020/245187

(56) References cited:
- WO-A1-2015/186114
- WO-A1-2017/011788
- WO-A2-2018/236814
- GB-A- 2 365 338
- MIKHAIL V MAKAROV ET AL: "Syntheses and chemical properties of [beta]-nicotinamide riboside and its analogues and derivatives", BEILSTEIN JOURNAL OF ORGANIC CHEMISTRY, vol. 15, 13 February 2019 (2019-02-13), GB, pages 401 - 430, XP055638913, ISSN: 1860-5397, DOI: 10.3762/bjoc.15.36
- JUDITH IROUD-GERBETANT ET AL: "A reduced form of nicotinamide riboside defines a new path for NAD+ biosynthesis and acts as an orally bioavailable NAD+ precursor", MOLECULAR METABOLISM, vol. 30, 3 October 2019 (2019-10-03), pages 192 - 202, XP055638821, ISSN: 2212-8778, DOI: https://doi.org/10.1016/j.molmet.2019.09.013

## Description

### FIELD OF THE INVENTION

The present invention provides compounds and compositions containing a reduced nicotinamide riboside, 1,4-dihydro-1-beta-D-ribofuranosyl-3-pyridinecarboxamide, for use in methods of prevention and/or treatment of acute kidney injury (AKI) and chronic kidney disease (CKD).

### BACKGROUND TO THE INVENTION

Acute kidney injury (AKI), also called acute renal failure, is more commonly reversible than chronic kidney failure (CKD). Nevertheless, AKI still carries a high mortality rate and is a critical risk factor for the development of chronic kidney disease (CKD). Acute kidney injury is particularly common in Intensive Care Unit (ICU) patients affecting more than 50% and is associated with increased mortality and morbidity.

CKD develops more slowly over time caused by a long-term disease, such as hypertension or diabetes, which slowly damages the kidneys and reduces their function over time.

AKI is still a major health burden with more than 13 million people affected each year. Despite all the advances in the field, the mortality of AKI remains very high estimated at 23.9% in adults and 13.8% in children (Alkhunaizi, 2018). If untreated, the resulting progression of AKI can lead to CKD or end stage renal disease (ESRD).

Currently, prevention of AKI is managed with timely resuscitation with fluids, vasopressors, and inotropic agents. Other than dialysis and renal transplantation, there are no known interventions that reliably improve survival, limit injury, or enhance recovery from CKD. WO2018/236814 discloses that nicotinamide riboside analogues may be used for treatment of kidney diseases. However, it does not specifically disclose that 1,4-dihydro-1-beta-D-ribofuranosyl-3-pyridinecarboxamide may be used to prevent or treat acute kidney disease and/or chronic kidney disease.

Therefore, there is an urgent unmet need to address kidney diseases, especially AKI with new compounds, compositions and methods of prevention and/or treatment of AKI and CKD.

### SUMMARY OF THE INVENTION

The invention is defined in the claims.

The present invention provides compounds and compositions comprising 1,4-dihydro-1-beta-D-ribofuranosyl-3-pyridinecarboxamide for use in methods of prevention and/or treatment of kidney diseases selected from acute kidney injury and chronic kidney disease.

In an embodiment, the composition is selected from the group consisting of: a pharmaceutical, food or beverage product, a food supplement, an oral nutritional supplement (ONS), a medical food, and combinations thereof.

As a precursor of NAD+ biosynthesis, reduced nicotinamide riboside, 1,4-dihydro-1-beta-D-ribofuranosyl-3-pyridinecarboxamide, can increase in NAD+ biosynthesis and provide one or more benefits to kidney function.

Also disclosed is a unit dosage form of a composition consisting of reduced nicotinamide riboside, 1,4-dihydro-1-beta-D-ribofuranosyl-3-pyridinecarboxamide, the unit dosage form contains an effective amount of the reduced nicotinamide riboside to increase NAD+ biosynthesis.

Yet another advantage of one or more of the embodiments of the invention consisting of reduced nicotinamide riboside, 1,4-dihydro-1-beta-D-ribofuranosyl-3-pyridinecarboxamide, is to reduce the progression of AKI to CKD.

### DETAILED DESCRIPTION OF THE INVENTION

### Definitions

All percentages expressed herein are by weight of the total weight of the composition unless expressed otherwise. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number.

All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used in this invention and the appended claims, the singular forms "a," "an" and "the" include plural referents unless the context clearly dictates otherwise. Thus, for example, reference to "a component" or "the component" includes two or more components.

The words "comprise," "comprises" and "comprising" are to be interpreted inclusively rather than exclusively. Likewise, the terms "include," "including" and "or" should all be construed to be inclusive, unless such a construction is clearly prohibited from the context. Nevertheless, the compositions disclosed herein may lack any element that is not specifically disclosed herein. Thus, a disclosure of an embodiment using the term "comprising" includes a disclosure of embodiments "consisting essentially of" and "consisting of" the components identified. Any embodiment disclosed herein can be combined with any other embodiment disclosed herein.

Where used herein, the terms "example" and "such as," particularly when followed by a listing of terms, are merely exemplary and illustrative and should not be deemed to be exclusive or comprehensive. As used herein, a condition "associated with" or "linked with" another condition means the conditions occur concurrently, preferably means that the conditions are caused by the same underlying condition, and most preferably means that one of the identified conditions is caused by the other identified condition.

The terms "food," "food product" and "food composition" mean a product or composition that is intended for ingestion by an individual such as a human and provides at least one nutrient to the individual. A food product typically includes at least one of a protein, a lipid, a carbohydrate and optionally includes one or more vitamins and minerals. The term "beverage" or "beverage product" means a liquid product or liquid composition that is intended to be ingested orally by an individual such as a human and provides at least one nutrient to the individual.

The compositions of the present disclosure, including the many embodiments described herein, can comprise, consist of, or consist essentially of the elements disclosed herein, as well as any additional or optional ingredients, components, or elements described herein or otherwise useful in a diet.

As used herein, the term "isolated" means removed from one or more other compounds or components with which the compound may otherwise be found, for example as found in nature. For example, "isolated" preferably means that the identified compound is separated from at least a portion of the cellular material with which it is typically found in nature. In an embodiment, an isolated compound is free from any other compound.

"Prevention" includes reduction of risk, incidence and/or severity of a condition or disorder. The terms "treatment," "treat" and "to alleviate" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The term does not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment," "treat" and "to alleviate" are also intended to include the potentiation or otherwise enhancement of one or more primary prophylactic or therapeutic measure. The terms "treatment," "treat" and "to alleviate" are further intended to include the dietary management of a disease or condition or the dietary management for prophylaxis or prevention a disease or condition. A treatment can be patient- or doctor-related.

The term "unit dosage form," as used herein, refers to physically discrete units suitable as unitary dosages for human and animal subjects, each unit containing a predetermined quantity of the composition disclosed herein in an amount sufficient to produce the desired effect, in association with a pharmaceutically acceptable diluent, carrier or vehicle. The specifications for the unit dosage form depend on the particular compounds employed, the effect to be achieved, and the pharmacodynamics associated with each compound in the host.

As used herein, an "effective amount" is an amount that prevents a deficiency, treats a disease or medical condition in an individual, or, more generally, reduces symptoms, manages progression of the disease, or provides a nutritional, physiological, or medical benefit to the individual. The relative terms "improve," "increase," "enhance," "promote" and the like refer to the effects of the composition disclosed herein, namely a composition comprising reduced nicotinamide riboside, relative to a composition not having nicotinamide riboside but otherwise identical. As used herein, "promoting" refers to enhancing or inducing relative to the level before administration of the composition disclosed herein.

As used herein "reduced nicotinamide riboside" may also be known as protonated nicotinamide riboside, dihydronicotinamide riboside, dihydro-1-beta-D-ribofuranosyl-3-pyridinecarboxamide, or 1-(beta-D-ribofuranosyl)-dihydronicotinamide. A description of the synthesis of reduced nicotinamide riboside is given in Example 1. The location of the protonation site can give rise to different forms of "reduced nicotinamide riboside". For example: 1,4-dihydro-1-beta-D-ribofuranosyl-3-pyridinecarboxamide; 1,2-dihydro-1-beta-D-ribofuranosyl-3-pyridinecarboxamide; and 1,6-dihydro-1-beta-D-ribofuranosyl-3-pyridinecarboxamide (Makarov and Migaud, 2019).

Classification of "Acute Kidney Injury" (AKI) is based on urine output and/or serum creatinine criteria. The most commonly used classifications of AKI are the "risk, injury, failure, loss of kidney function, and end-stage kidney disease" (RIFLE) and the Acute Kidney Injury Network (AKIN) classifications (Alkhunaizi, 2018). Recent consensus of the definition of AKI is now defined as an abrupt reduction in renal function (within 48 h) based on an increase in serum creatinine level of more than or equal to 0.3 mg/dL (≥26.4 µmol/L), a percentage increase in serum creatinine of more than or equal to 50% (1.5-fold from baseline), or a reduction in urine output (documented oliguria of less than 0.5 mL/kg/h for more than 6 h) or a combination of these factors (Alkunaizi, 2018).

Some factors responsible for the pathophysiology of AKI include: renal microvasculature damage and inflammation. Renal microvasculature is important because adequate oxygen delivery is crucial for the production of mitochondrial adenosine triphosphate (ATP), nitric oxide (NO), and reactive oxygen species (ROS) necessary for homeostatic control of renal function. Inflammation, for example related to sepsis plays a major role in the pathophysiology of AKI resulting from ischemia leads to activation of cytokines and inflammatory pathways resulting in a loss in renal function, decreases renal injury, cell death, and long-term fibrosis.

"Chronic Kidney Disease" (CKD) is the end stage which over time can result in complete loss of kidney function if not treated at an earlier stage. As it often progresses undetected secondary to other diseases or conditions such as diabetes, glomerulonephritis or hypertension generally only detected as an increase in serum creatinine or protein in the urine. As the kidney function decreases, urea accumulates leading to azotemia and ultimately uremia. Potassium accumulates in the blood potentially leading to hyperkalemia. Hyperphosphatemia, due to reduced phosphate excretion, follows the decrease in glomerular filtration. Hyperphosphatemia is associated with increased cardiovascular risk.

### Embodiments

The invention is defined in the claims.

The present invention provides compounds and compositions consisting of reduced nicotinamide riboside in the form of 1,4-dihydro-1-beta-D-ribofuranosyl-3-pyridinecarboxamide. Another aspect of the present invention is a unit dosage form of a composition consisting of 1,4-dihydro-1-beta-D-ribofuranosyl-3-pyridinecarboxamide and the unit dosage form contains the 1,4-dihydro-1-beta-D-ribofuranosyl-3-pyridinecarboxamide in an amount effective for a subject in need thereof.

The increase in NAD⁺ biosynthesis can provide one or more benefits to the individual, for example a human (e.g., a human undergoing medical treatment), a pet or a horse (e.g., a pet or horse undergoing medical treatment), or cattle or poultry (e.g., cattle or poultry being used in agriculture) with respect to prevention or treatment of kidney disease.

For non-human mammals such as rodents, some embodiments comprise administering an amount of the composition that provides 1.0 mg to 1.0 g of the reduced nicotinamide riboside / kg of body weight of the non-human mammal, preferably 10 mg to 500 mg of the reduced nicotinamide riboside / kg of body weight of the non-human mammal, more preferably 25 mg to 400 mg of the reduced nicotinamide riboside / kg of body weight of the mammal, most preferably 50 mg to 300 mg of the reduced nicotinamide riboside / kg of body weight of the non-human mammal.

For humans, some embodiments comprise administering an amount of the composition that provides 1.0 mg to 10.0 g of the reduced nicotinamide riboside / kg of body weight of the human, preferably 10 mg to 5.0 g of the reduced nicotinamide riboside / kg of body weight of the human, more preferably 50 mg to 2.0 g of the reduced nicotinamide riboside / kg of body weight of the human, most preferably 100 mg to 1.0 g of the reduced nicotinamide riboside / kg of body weight of the human.

In some embodiments, at least a portion of the reduced nicotinamide riboside is isolated from natural plant sources. Additionally or alternatively, at least a portion of reduced nicotinamide riboside can be chemically synthesized. For example, according to Example 1 described below.

As used herein, a "composition consisting essentially of reduced nicotinamide riboside" contains reduced nicotinamide riboside in the form of 1,4-dihydro-1-beta-D-ribofuranosyl-3-pyridinecarboxamide and does not include, or is substantially free of, or completely free of, any additional compound that affects NAD+ production other than the "reduced nicotinamide riboside". In a particular non-limiting embodiment, the composition consists of the reduced nicotinamide riboside in the form of 1,4-dihydro-1-beta-D-ribofuranosyl-3-pyridinecarboxamide and an excipient or one or more excipients.

In some embodiments, the composition consisting essentially of reduced nicotinamide riboside in the form of 1,4-dihydro-1-beta-D-ribofuranosyl-3-pyridinecarboxamide is optionally substantially free or completely free of other NAD+ precursors, such as nicotinamide riboside.

As used herein, "substantially free" means that any of the other compounds present in the composition is no greater than 1.0 wt.% relative to the amount of reduced nicotinamide riboside, preferably no greater than 0.1 wt.% relative to the amount of reduced nicotinamide riboside, more preferably no greater than 0.01 wt.% relative to the amount of reduced nicotinamide riboside, most preferably no greater than 0.001 wt.% relative to the amount of reduced nicotinamide riboside.

The 1,4-dihydro-1-beta-D-ribofuranosyl-3-pyridinecarboxamide and compositions thereof can promote the increase of intracellular levels of NAD⁺ in cells and tissues for improving cell and tissue survival and overall cell and tissue health, for example, in kidney cells and tissues.

Nicotinamide adenine dinucleotide (NAD+) is considered a coenzyme, and essential cofactor in cellular redox reactions to produce energy. It plays critical roles in energy metabolism, as the oxidation of NADH to NAD+ facilitates hydride-transfer, and consequently ATP generation through mitochondrial oxidative phosphorylation. It also acts as a degradation substrate for multiple enzymes (Canto,C. et al. 2015; Imai,S. et al. 2000; Chambon,P. et al. 1963; Lee, H.C. et al. 1991).

Mammalian organisms can synthesize NAD+ from four different sources. First, NAD+ can be obtained from tryptophan through the 10-step de novo pathway. Secondly, Nicotinic acid (NA) can also be transformed into NAD+ through the 3-step Preiss-Handler path, which converges with the de novo pathway. Thirdly, intracellular NAD+ salvage pathway from nicotinamide (NAM) constitutes the main path by which cells build NAD+, and occurs through a 2-step reaction in which NAM is first transformed into NAM-mononucleotide (NMN) via the catalytic activity of the NAM-phosphoribosyltransferase (NAMPT) and then converted to NAD+ via NMN adenylyltransferase (NMNAT) enzymes. Finally, Nicotinamide Riboside (NR) constitutes yet a fourth path to NAD+, characterized by the initial phosphorylation of NR into NMN by NR kinases (NRKs)( Breganowski,P. et al.; 2004).

Five molecules previously have been known to act as direct extracellular NAD+ precursors: tryptophan, nicotinic acid (NA), nicotinamide (NAM), nicotinic acid riboside (NaR) and nicotinamide riboside (NR). The present invention, discloses a new molecule that can act as an extracellular NAD+ precursor, reduced nicotinomide riboside (NRH). The reduction of the NR molecule to NRH confers it not only a much stronger capacity to increase intracellular NAD+ levels, but also a different selectivity in terms of its cellular use.

The present invention relates to NRH, in the form of 1,4-dihydro-1-beta-D-ribofuranosyl-3-pyridinecarboxamide, a new molecule which can act as an NAD+ precursor. This reduced form of NR, which displays an unprecedented ability to increase NAD+ and has the advantage of being more potent and faster than nicotinamide riboside (NR). NRH utilizes a different pathway than NR to synthesize NAD+, which is NRK independent. The present invention demonstrates that NRH is protected against degradation in plasma and can be detected in circulation after oral administration. These advantages of the invention support its therapeutic efficacy.

In each of the compositions and methods disclosed herein, the composition is preferably a food or beverage product, including food additives, food ingredients, functional foods, dietary supplements, medical foods, nutraceuticals, oral nutritional supplements (ONS) or food supplements.

The composition can be administered at least one day per week, preferably at least two days per week, more preferably at least three or four days per week (e.g., every other day), most preferably at least five days per week, six days per week, or seven days per week. The time period of administration can be at least one week, preferably at least one month, more preferably at least two months, most preferably at least three months, for example at least four months. In some embodiments, dosing is at least daily; for example, a subject may receive one or more doses daily, in an embodiment a plurality of doses per day. In some embodiments, the administration continues for the remaining life of the individual. In other embodiments, the administration occurs until no detectable symptoms of the medical condition remain. In specific embodiments, the administration occurs until a detectable improvement of at least one symptom occurs and, in further cases, continues to remain ameliorated.

The compositions disclosed herein may be administered to the subject enterally, e.g., orally, or parenterally. Non-limiting examples of parenteral administration include intravenously, intramuscularly, intraperitoneally, subcutaneously, intraarticularly, intrasynovially, intraocularly, intrathecally, topically, and inhalation. As such, non-limiting examples of the form of the composition include natural foods, processed foods, natural juices, concentrates and extracts, injectable solutions, microcapsules, nano-capsules, liposomes, plasters, inhalation forms, nose sprays, nosedrops, eyedrops, sublingual tablets, and sustained-release preparations.

The compositions disclosed herein can use any of a variety of formulations for therapeutic administration. More particularly, pharmaceutical compositions can comprise appropriate pharmaceutically acceptable carriers or diluents and may be formulated into preparations in solid, semi-solid, liquid or gaseous forms, such as tablets, capsules, powders, granules, ointments, solutions, suppositories, injections, inhalants, gels, microspheres, and aerosols. As embodiments, the administration occurs until a detectable improvement of at least one symptom occurs and, in further cases, continues to remain ameliorated.

The compositions disclosed herein may be administered to the subject enterally, e.g., orally, or parenterally. Non-limiting examples of parenteral administration include intravenously, intramuscularly, intraperitoneally, subcutaneously, intraarticularly, intrasynovially, intraocularly, intrathecally, topically, and inhalation. As such, non-limiting examples of the form of the composition include natural foods, processed foods, natural juices, concentrates and extracts, injectable solutions, microcapsules, nano-capsules, liposomes, plasters, inhalation forms, nose sprays, nosedrops, eyedrops, sublingual tablets, and sustained-release preparations.

The compositions disclosed herein can use any of a variety of formulations for therapeutic administration. More particularly, pharmaceutical compositions can comprise appropriate pharmaceutically acceptable carriers or diluents and may be formulated into preparations in solid, semi-solid, liquid or gaseous forms, such as tablets, capsules, powders, granules, ointments, solutions, suppositories, injections, inhalants, gels, microspheres, and aerosols. As such, administration of the composition can be achieved in various ways, including oral, buccal, rectal, parenteral, intraperitoneal, intradermal, transdermal, and intratracheal administration. The active agent may be systemic after administration or may be localized by the use of regional administration, intramural administration, or use of an implant that acts to retain the active dose at the site of implantation.

In pharmaceutical dosage forms, the compounds may be administered as their pharmaceutically acceptable salts. They may also be used in appropriate association with other pharmaceutically active compounds. The following methods and excipients are merely exemplary and are in no way limiting.

For oral preparations, the compounds can be used alone or in combination with appropriate additives to make tablets, powders, granules or capsules, for example, with conventional additives, such as lactose, mannitol, corn starch or potato starch; with binders, such as crystalline cellulose, cellulose functional derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The compounds can be formulated into preparations for injections by dissolving, suspending or emulsifying them in an aqueous or non-aqueous solvent, such as vegetable or other similar oils, synthetic aliphatic acid glycerides, esters of higher aliphatic acids or propylene glycol; and if desired, with conventional, additives such as solubilizers, isotonic agents, suspending agents, emulsifying agents, stabilizers and preservatives.

The compounds can be utilized in an aerosol formulation to be administered by inhalation. For example, the compounds can be formulated into pressurized acceptable propellants such as dichlorodifluoromethane, propane, nitrogen and the like.

Furthermore, the compounds can be made into suppositories by mixing with a variety of bases such as emulsifying bases or water-soluble bases. The compounds can be administered rectally by a suppository. The suppository can include a vehicle such as cocoa butter, carbowaxes and polyethylene glycols, which melt at body temperature, yet are solidified at room temperature.

Unit dosage forms for oral or rectal administration such as syrups, elixirs, and suspensions may be provided wherein each dosage unit, for example, teaspoonful, tablespoonful, tablet or suppository, contains a predetermined amount of the composition. Similarly, unit dosage forms for injection or intravenous administration may comprise the compounds in a composition as a solution in sterile water, normal saline or another pharmaceutically acceptable carrier, wherein each dosage unit, for example, mL or L, contains a predetermined amount of the composition containing one or more of the compounds.

Compositions intended for a non-human animal include food compositions to supply the necessary dietary requirements for an animal, animal treats (e.g., biscuits), and/or dietary supplements. The compositions may be a dry composition (e.g., kibble), semi-moist composition, wet composition, or any mixture thereof. In one embodiment, the composition is a dietary supplement such as a gravy, drinking water, beverage, yogurt, powder, granule, paste, suspension, chew, morsel, treat, snack, pellet, pill, capsule, tablet, or any other suitable delivery form. The dietary supplement can comprise a high concentration of the UFA and NORC, and B vitamins and antioxidants. This permits the supplement to be administered to the animal in small amounts, or in the alternative, can be diluted before administration to an animal. The dietary supplement may require admixing, or can be admixed with water or other diluent prior to administration to the animal.

### REFERENCES

Alkhunaizi, A. 2018, Ch.2 - Acute Kidney Injury, in "Aspect of Continuous Renal Replacement Therapy", 2018, pgs. 1-29, Intech Open.
Bieganowski, P. and C. Brenner, 2004. Discoveries of nicotinamide riboside as a nutrient and conserved NRK genes establish a Preiss-Handler independent route to NAD+ in fungi and humans. Cell. 117(4): 495-502.
Canto, C., K.J. Menzies, and J. Auwerx, 2015. NAD(+) Metabolism and the Control of Energy Homeostasis: A Balancing Act between Mitochondria and the Nucleus. Cell Metab. 22(1): 31-53.
Cao, Zemin; Cooper, Mark E. 2011, Pathogenesis of Diabetic Neuropathy; Journal of Diabetes Investigation, Vol.2, Issue 1, pgs. 243-247.
Chambon, P., J.D. Weill, and P. Mandel, 1963. Nicotinamide mononucleotide activation of new DNA-dependent polyadenylic acid synthesizing nuclear enzyme. Biochem Biophys Res Commun. 1139-43.
Imai, S., C.M. Armstrong, M. Kaeberlein, and L. Guarente, 2000. Transcriptional silencing and longevity protein Sir2 is an NAD-dependent histone deacetylase. Nature. 403(6771): 795-800.
Lee, H.C. and R. Aarhus, 1991. ADP-ribosyl cyclase: an enzyme that cyclizes NAD+ into a calcium-mobilizing metabolite. Cell Regul. 2(3): 203-9.
Makarov, M. and M. Migaud, 2019. Syntheses and chemical properties of β-nicotinamide riboside and its analogues and derivatives. Beilstein J. Org. Chem. 15: 401-430

### DESCRIPTION OF FIGURES

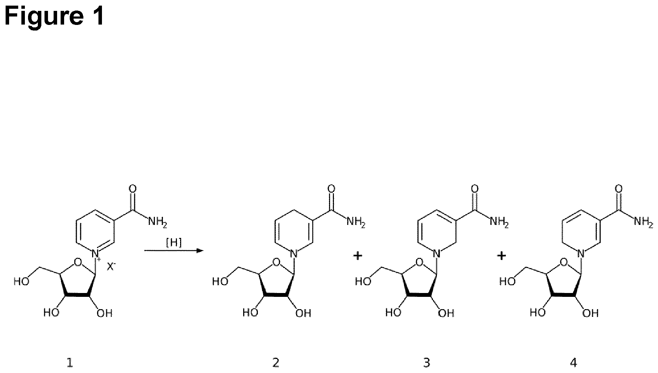

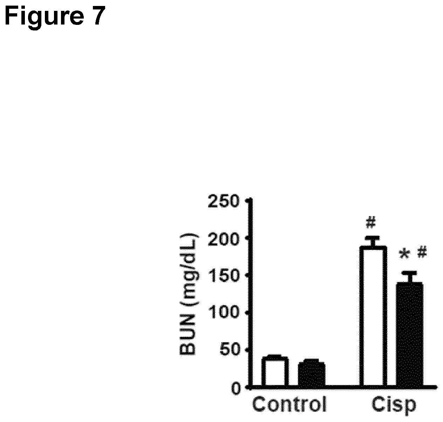

**Figure 1****. Chemical structure of nicotinamide riboside in its oxidized (NR) and reduced (NRH) forms**
   1: 1-b-D-ribofuranosyl-3-pyridinecarboxamide salt
   2: 1,4-dihydro-1-b-D-ribofuranosyl-3-pyridinecarboxamide
   3: 1,2-dihydro-1-b-D-ribofuranosyl-3-pyridinecarboxamide
   4: 1,6-dihydro-1-b-D-ribofuranosyl-3-pyridinecarboxamide
   X⁻: anion (e.g. triflate)
**Figure 2****. Dose-response experiments revealed that NRH could significantly increase NAD+ better than NR**
   Starting at levels at a concentration of 10 µM, NRH achieved similar increases in intracellular NAD+ levels to those reached with NR at 50-fold higher concentrations. NRH achieved maximal effects on NAD+ synthesis around the millimolar range, managing to increase intracellular NAD+ levels by more than 10-fold.

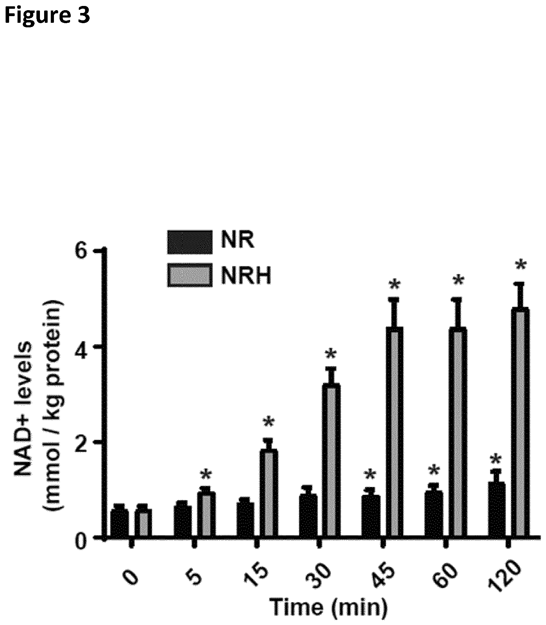

**Figure 3****. NHR acts rapidly after 5 minutes from treatment.**
   NRH actions were also extremely fast, as significant increases in NAD+ levels were observed within 5 minutes after NRH treatment. Peak levels of NAD+ were achieved between 45 minutes and 1 h after treatment.

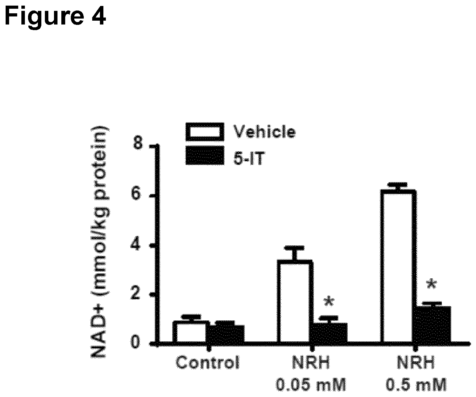

**Figure 4****. NRH leads to NAD+ biosynthesis through an adenosine kinase dependent path.**
   AML12 cells were treated with an adenosine kinase inhibitor (5-IT; 10 mM) for 1 hour prior to NRH treatment at the doses indicated. Then, 1 hour later, acidic extracts were obtained to measure NAD⁺ levels. All values in the figure are expressed as mean +/- SEM of 3 independent experiments. * indicates statistical difference at p< 0.05 vs. the respective vehicle treated group.

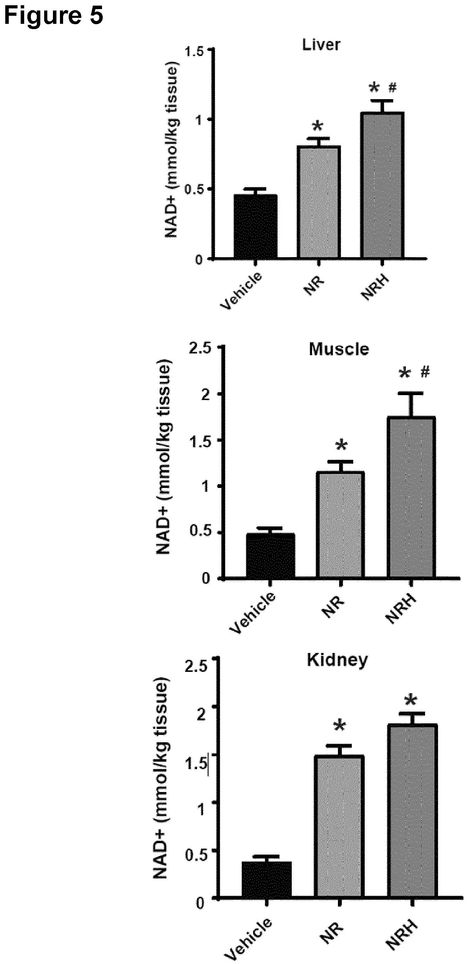

**Figure 5****. NRH is an orally active NAD+ precursor in liver, muscle and kidney..**
   8 week-old C57BI/6NTac mice were orally gavaged with either saline (as vehicle), NR (500 mg/kg) or NRH (500 mg/kg). After 1 hour, liver, skeletal muscle and kidney NAD⁺ levels were evaluated. All results are expressed as mean +/-SEM of n=5 mice per group. * indicates statistical difference at p<0.05 vs. vs. saline-treated mice. # indicates statistical difference at p<0.05 vs. NR treated mice.

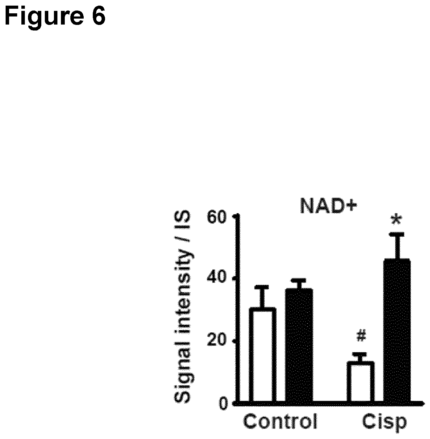

**Figure 6****. NRH protects against cisplatin-induced renal NAD⁺ depletion.**
   8 week old C57BI/6NTac mice were intraperitoneally injected with either saline (control) or cisplatin (Cisp, 20 mg/kg). Simultaneously, mice were intraperitoneally injected with PBS (as vehicle) or NRH (250 mg/kg). PBS or NRH was injected at 24, 48 and 72 h after the initiation of the experiment. Kidneys of the mice were harvested, 4 h after the last injection, and NAD+ levels were analyzed in the kidney through mass spectrometry. All results are expressed as mean +/-SEM of n=5 mice per group. * indicates statistical difference at p<0.05 vs. vs. respective saline-treated mice
**Figure 7****. NRH decreases blood urea nitrogen levels in cisplatin-induced acute kidney injury.** 8 week old C57BI/6NTac mice were intraperitoneally injected with either saline (control) or cisplatin (Cisp, 20 mg/kg). Simultaneously, mice were intraperitoneally injected or not with PBS (as vehicle) or NRH (250 mg/kg) at 24, 48 and 72 h after the initiation of the experiment. Mice were sacrificed 4 h after the last injection, and plasma was collected to measure blood urea nitrogen levels. All results are expressed as mean +/-SEM of n=5 mice per group. * indicates statistical difference at p<0.05 vs. vs. respective saline-treated mice

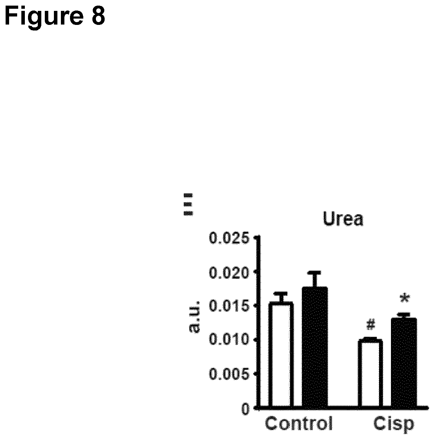

**Figure 8****. NRH recovers urea levels in urine in a model of acute kidney injury.**
   8 week old C57BI/6NTac mice were intraperitoneally injected with either saline (control) or cisplatin (Cisp, 20 mg/kg). Simultaneously, mice were intraperitoneally injected or not with PBS (as vehicle) or NRH (250 mg/kg). Urine was collected 24 h later to evaluate urea levels by NMR. All results are expressed as mean +/-SEM of n=5 mice per group. * indicates statistical difference at p<0.05 vs. vs. respective saline-treated mice.

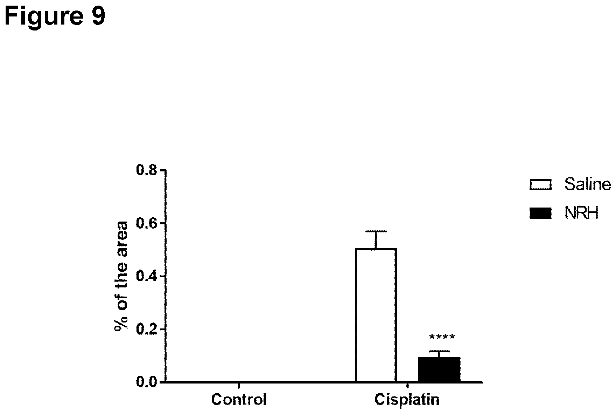

**Figure 9****. NRH protects against cisplatin-induced renal cell apoptosis.**
   8 week old C57BI/6NTac mice were intraperitoneally injected with either saline (control) or cisplatin (Cisp, 20 mg/kg). Simultaneously, mice were intraperitoneally injected or not with PBS (as vehicle) or NRH (250 mg/kg). PBS or NRH were then injected at 24, 48 and 72 h after the initiation of the experiment. Mice were sacrificed 4 h after the last injection, and kidneys were fixed in OCT and used for immunohistochemistry against cleaved caspase 3. The cleaved caspase 3 staining was then quantified against the total area, using 10 images per mouse, 5 mice per group. All results are expressed as mean +/-SEM. * indicates statistical difference at p<0.05 vs. vs. respective saline-treated mice.

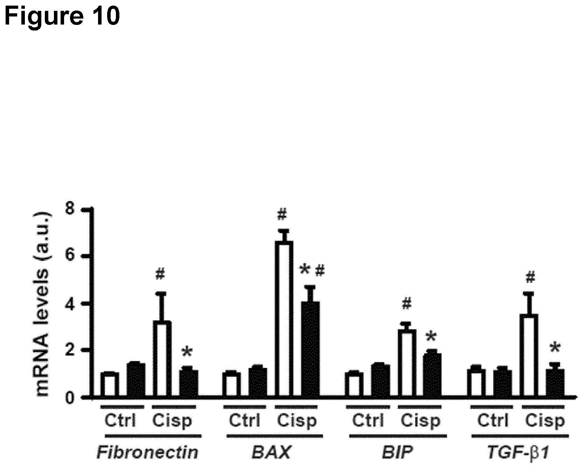

**Figure 10****. NRH protects against cisplatin induced ER stress and apoptosis.**
   8 week old C57BI/6NTac mice were intraperitoneally injected with either saline (control) or cisplatin (Cisp, 20 mg/kg). Simultaneously, mice were intraperitoneally injected or not with PBS (as vehicle) or NRH (250 mg/kg). PBS or NRH were then injected at 24, 48 and 72 h after the initiation of the experiment. 4 h after the last injection, and kidneys were obtained to isolate mRNA and analyze by qPCR makers of renal dysfunction (TGF-b1), glomerular dysfunction (fibronectin), apoptosis (BAX) and ER stress (BIP). All values are expressed as mean +/- SEM of n=4 mice per group. * indicates statistical difference at p<0.05 vs. respective vehicle-injected mice. # indicates statistical difference at p<0.05 vs. the respective mice in the control group.

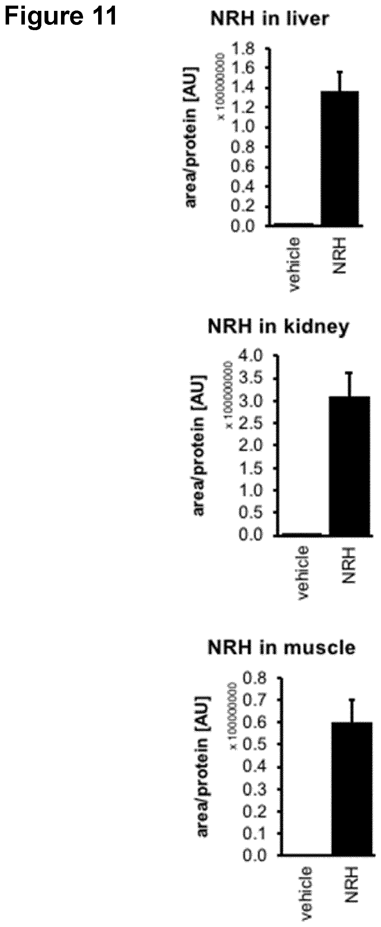

**Figure 11****. NRH is found intact in mice tissues after administration.**
   8 week-old C57BI/6NTac mice were orally gavaged with either saline (as vehicle), and NRH (250 mg/kg). After 2 hours, liver, skeletal muscle and kidney NRH levels were evaluated. All results are expressed as mean +/-SEM of n=4 mice per group, as areas under the signal by LC-MS analysis, corrected by total protein amount of tissue.

### EXAMPLES

### Example 1: Synthesis of the reduced form of nicotinamide riboside (NRH)

Reduced nicotinamide riboside (NRH) was obtained from NR (1) by reduction of pyridinium salts (for example, triflate) to dihydropyridines (1,2-, 1,4-, and 1,6-dihydropyridines) as shown below 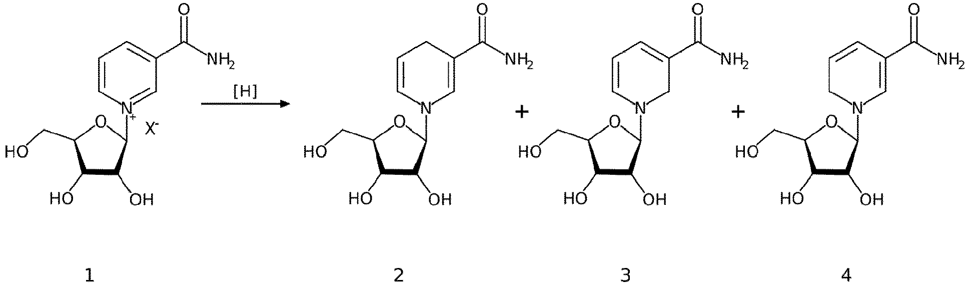
1: 1-b-D-ribofuranosyl-3-pyridinecarboxamide salt
2: 1,4-dihydro-1-beta-D-ribofuranosyl-3-pyridinecarboxamide
3: 1,2-dihydro-1-beta-D-ribofuranosyl-3-pyridinecarboxamide
4: 1,6-dihydro-1-beta-D-ribofuranosyl-3-pyridinecarboxamide
X⁻: anion (e.g. triflate)

Sodium borohydride (NaBH₄) and sodium dithionite (Na₂S₂O₄) were used as reducing agents for *N*-substituted pyridinium derivatives. Regioselectivity of reducing agents differ, leading to either only one dihydropyridine or a mixture of all 3 isomers in different proportions (2,3,4).

Dithionate reduction of pyridinium salts, carrying electron withdrawing substituents in positions 3 and 5, yielded almost exclusively 1,4-dihydropyridine products. The reduction was made in mild conditions (e.g. in aqueous sodium bicarbonate or potassium phosphate dibasic medium), due to instability of the reduced products in acidic media. To perform the reduction, hydroxyl groups in the ribofuranose moiety were protected with either benzyl or acetyl substituents. Deprotection was then be done by sodium hydroxide in methanol under ball mill conditions, after reduction.

### Example 2: Measurement of NRH and other NAD+ related metabolites in biological samples

Levels of NRH and other NAD-related metabolites in biological samples were obtained by using a cold liquid-liquid extraction using a mixture of methanol:water:chloroform in 5:3:5 (v/v), from which the polar phase was recovered for hydrophilic interaction ultra-high performance liquid chromatography mass spectrometry (UHPLC-MS) analysis. The UHPLC consisted of a binary pump, a cooled autosampler, and a column oven (DIONEX Ultimate 3000 UHPLC+ Focused, Thermo Scientific), connected to a triple quadrupole spectrometer (TSQ Vantage, Thermo Scientific) equipped with a heated electrospray ionisation (H-ESI) source. Of each sample, 2 µL were injected into the analytical column (2.1 mm x 150 mm, 5 µm pore size, 200 Å HILICON iHILIC^{®}-Fusion(P)), guarded by a pre-column (2.1 mm x 20 mm, 200 Å HILICON iHILIC^{®}-Fusion(P) Guard Kit) operating at 35 °C. The mobile phase (10 mM ammonium acetate at pH 9, A, and acetonitrile, B) was pumped at 0.25 mL/min flow rate over a linear gradient of decreasing organic solvent (0.5-16 min, 90-25% B), followed by re-equilibration for a total run time of 30 min. The MS operated in positive mode at 3500 V with multiple reaction monitoring (MRM). The software Xcalibur v4.1.31.9 (Thermo Scientific) was used for instrument control, data acquisition and processing. Retention time and mass detection was confirmed by authentic standards.

Structure elucidation of the used NRH for biological studies was confirmed by nuclear magnetic resonance (NMR).

### Example 3: NRH is a potent NAD+ precursor

AML12 hepatocytes were treated with NRH, and it was observed that the ability of NRH to increase intracellular NAD+ was superior to that of NR.

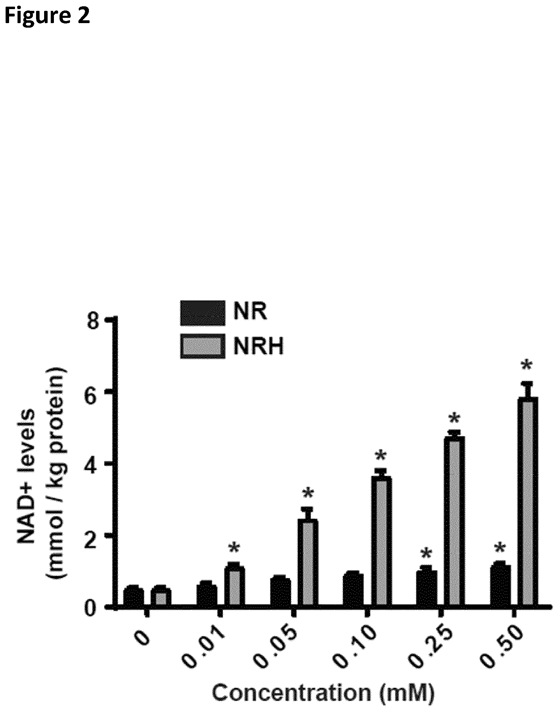

Dose-response experiments revealed that NRH could significantly increase NAD+ levels at a concentration of 10 µM (Figure 2). Even at such relatively low dose, NRH achieved similar increases in intracellular NAD+ levels to those reached with NR at 50-fold higher concentrations. NRH achieved maximal effects on NAD+ synthesis around the millimolar range, managing to increase intracellular NAD+ levels by more than 10-fold.

NRH actions were also extremely fast (Figure 3), as significant increases in NAD+ levels were observed within 5 minutes after NRH treatment. Peak levels of NAD+ were achieved between 45 minutes and 1 h after treatment, as also occurred with NR.

The ability of NRH to potently increase NAD+ was tested as well in other cell type models. NRH treatment highly elevated NAD+ levels in C2C12 myotubes, INS1-cells and 3T3 fibroblasts, supporting the notion that NRH metabolism is widely conserved among different cell types.

### Example 4: Pathway of NRH-induced NAD+ synthesis

A path in which NRH would be converted to NMNH, then to NADH and this would be finally oxidized to NAD+. Accordingly, NRH and NMNH could be detected intracellularly 5 minutes after NRH, but not NR, treatment. Interestingly, NRH treatment also led to an increase in intracellular NR and NMN, greater than that triggered by NR itself, opening the possibility that NRH could synthesize NAD+ by being oxidized to NR, using then the canonical NRK/NMNAT path.

In order to understand the exact path by which NRH synthesizes NAD+, we initially evaluated whether NRH, could be transported into the cell by equilibrative nucleoside transporters (ENTs). Confirming this possibility, NRH largely lost its capacity as an extracellular NAD+ precursor in the presence of an agent blocking ENT-mediated transport, such as S-(4-nitrobenzyl)-6-thioinosine (NBTI). Nevertheless, a substantial action of NRH remained even after ENT blockage, suggesting that NRH might be able to enter the cell through additional transporters.

The action of NRH was also NAMPT-independent, based on experiments using FK866, a NAMPT inhibitor. If NRH led to NAD+ synthesis via the formation of NMNH, this hypothetical path would require the phosphorylation of NRH into NMNH. Given the essential and rate-limiting role of NRK1 in NR phosphorylation, we wondered whether the ability of NRH to boost NAD+ levels was NRK1 dependent. To answer this question, we evaluated NRH action in primary hepatocytes from either control or NRK1 knockout (NRK1KO) mice. While after 1 hour of treatment NR failed to increase NAD+ levels in NRK1KO derived primary hepatocytes, NRH action was not affected by NRK1 deficiency. These results indicate that NRH action is NRK1 independent. Further, they rule out the possibility that NRH-induced NAD+ transport is driven by NRH oxidation into NR.

Considering the molecular structure of NRH, we reasoned that an alternative nucleoside kinase could be responsible for the phosphorylation of NRH. Confirming this expectation, the adenosine kinase (AK) inhibitor 5-iodotubercidin (5-IT) fully ablated the action of NRH. The role of AK in NRH-mediated NAD+ synthesis was confirmed using a second, structurally different, AK inhibitor, ABT-702. Metabolomic analyses further confirmed that upon inhibition of AK, the generation of NMNH, NADH and NAD+ was fully blunted, even if NRH was effectively entering the cell. Interestingly, 5-IT treatment also prevented the formation of NR and NMN after NRH treatment.

This indicates that the occurrence of NR after NRH treatment cannot be attributed simply to direct NRH intracellular oxidation to NR. As a whole, these experiments depict adenosine kinase as the enzymatic activity catalyzing the conversion of NRH into NMNH, initiating this way the transformation into NAD+.

As a follow-up step, NMNAT enzymes could catalyze the transition from NMNH to NADH. Accordingly, the use of gallotannin as a NMNAT inhibitor largely compromised NAD+ synthesis after NRH treatment. Yet, part of the NRH action remained after gallotannin treatment when NRH was used at maximal doses. However, NRH action was totally blocked by gallotannin at submaximal doses, suggesting that the remaining effect at 0.5 mM could be attributed to incomplete inhibition of NMNAT activity by gallotannin. Altogether, these results indicate that adenosine kinase and NMNATs vertebrate the path by which NRH leads to NAD+ synthesis via NADH.

### Example 5: NRH is detectable in circulation after IP injection

NR degradation to NAM has been proposed as a limitation for its pharmacological efficacy. To evaluate whether NRH was also susceptible to degradation to NAM, we spiked NRH or NR in isolated mouse plasma. After 2 h of incubation, NR levels decayed in plasma, in parallel to an increase in NAM. In contrast, NAM was not generated from NRH, as its levels remained stable during the 2 h test. We also tested the stability of NRH in other matrixes. Given our previous experiments in cultured cells, we verified that NRH did not degrade to NAM in FBS supplemented media, as occurs with NR. Finally, we also certified NRH stability in water (pH=7, at room temperature) for 48 h.

The above results prompted us to test whether NRH could act as an effective NAD+ precursor in vivo. For this, we first intraperitoneally (IP) injected mice with either NR or NRH (500 mg/kg). After 1 h, both compounds increased NAD+ levels in liver (Figure 5), muscle and kidney. As expected, NAM levels were highly increased in circulation upon NR administration, while only a very mild increase was observed with NRH. Importantly, NRH was detectable in circulation after IP injection.

To our surprise, NR was detectable in circulation after NRH treatment at much higher levels than those detected after NR injection itself. Given that NRH incubation in isolated plasma did not lead to NR production, the appearance of NR might be consequent to intracellular production and release to circulation. Similarly, the residual appearance of NAM after NRH treatment might be explained by the degradation of released NR or by the release of intracellular NAM as a product of NAD+ degradation, as NRH did not significantly alter NAM levels when incubated in isolated plasma.

### Example 6: NRH is detectable after oral administration as an orally bioavailable NAD+ precursor that overcomes direct degradation in plasma

Oral administration of NRH led to very similar results to those observed after IP administration. First, NRH had a more potent effect on hepatic NAD+ levels than NR. NRH was detectable in plasma 1 h after oral administration. In contrast, NR levels were undetectable at 1 h after NR administration. As expected, NR treatment led to large increases in circulating NAM, which where -4-fold higher than those observed after NRH treatment. Quantification measurements revealed that after oral gavage, NRH concentration in plasma reached 11.16 ± 1.74 micromolar, which is enough to effectively drive NAD+ synthesis. These results illustrate that NRH is a potent orally bioavailable NAD+ precursor that overcomes direct degradation to NAM in plasma.

### Example 7: NRH protects against cisplatin-induced acute kidney injury

To evaluate the potential therapeutic actions of NRH on a model of acute kidney injury (AKI), 8-week old mice were injected with either vehicle or cisplatin (20 mg/kg). Mice were then repeatedly injected with either vehicle or NRH (250 mg/kg) at 0, 24, 48 and 72 hrs after cisplatin injection. Kidneys were harvested 4 hrs after the last NRH injection.

Cisplatin treatment led to a decrease in renal NAD⁺ (Figure 6) and NADH levels in parallel to an increase in NAM and methyl-NAM levels. This was also reflected in the levels of methylated-oxidized NAM metabolites in urine, N-methyl-2-pyridone-5-carboxamide (Me2PY) or N-methyl-4-pyridone-5-carboxamide (Me4PY). This suggests that cisplatin increases the rate of NAD⁺ degradation to NAM, probably due to the activation of PARP enzymes, NRH supplementation prevented the drop in renal NAD⁺ and NADH levels induced by cisplatin. We did not observe higher NAD⁺ levels in kidney 4 h after NRH supplementation. This could be due to a rather high NAD⁺ turnover in the kidney upon sustained NAD⁺ consumption by cisplatin induced DNA damage, as increased NAD⁺ were observed at shorter time frames. Interestingly, NRH further increased methyl-NAM levels in kidney and Me2PY and Me4PY levels in urine, indicating that NRH can sustain NAD⁺ production and further allow the activation of NAD⁺ consuming enzymes. PARP activity was higher in cisplatin mice treated with NRH. Overall, these data indicate that the genotoxic action of cisplatin leads to PARP activation and NAD⁺ depletion, to the point that decreased NAD⁺ levels might limit PARP activity. NRH supplementation allows sustaining NAD⁺ consuming-enzymes activities by preserving NAD⁺ levels.

NRH injections also alleviated the increases in blood urea nitrogen (BUN) triggered by cisplatin-related kidney damage (Figure 7). Concomitantly, urea levels in urine were higher upon NRH treatment, further suggesting a better renal function (Figure 8). At the histological level, NRH treatment did not lead to any major ultrastructural change in kidney. Cisplatin treatment led to marked alteration in kidney structure, including increased tubular necrosis, glomeruli dilatation, inflammation and a cast formation. These features were largely prevented by NRH treatment including a major decrease in the presence of kidney casts (Figure 9). In agreement with this, NRH also prevented cisplatin-induced increases in makers of glomerular dysfunction (fibronectin), apoptosis (BAX) and ER stress (BIP) (Figure 10). This could be due to the ability of NRH to prevent the increase in TGF-β1 expression induced by cisplatin, which is a key agent triggering apoptosis and fibrogenesis in the kidney, both being critical factors in the development of kidney disease.

### Example 8: NRH is found intact in liver, kidney and muscle after oral administration.

NRH is not only found in circulation but it was also found intact, in high levels, in mice liver, kidney and muscle 2 hours after gavage (Figure 11). This indicates that oral administration of NRH allows for efficient biodistribution in target tissues.

## Claims

1. 1,4-dihydro-1-beta-D-ribofuranosyl-3-pyridinecarboxamide for use in preventing and/or treating kidney diseases and conditions selected from acute kidney injury and chronic kidney disease, in a subject, comprising delivering to the subject in need an effective unit dose form of 1,4-dihydro-1-beta-D-ribofuranosyl-3-pyridinecarboxamide.

2. Composition comprising 1,4-dihydro-1-beta-D-ribofuranosyl-3-pyridinecarboxamide for use to prevent and/or treat kidney disease selected from acute kidney injury and chronic kidney disease.

3. Composition for use according to claim 2 which is selected from the group consisting of: a pharmaceutical, food or beverage product, a food supplement, an oral nutritional supplement (ONS), a medical food, and combinations thereof.

4. Composition for use according to claim 2 or 3 wherein the subject is a human.

## Patentansprüche

1. 1,4-Dihydro-1-beta-D-ribofuranosyl-3-pyridincarboxamid zur Verwendung bei einer Vorbeugung und/oder Behandlung von Nierenerkrankungen und -zuständen, die aus akutem Nierenversagen und chronischer Nierenerkrankung ausgewählt sind, in einem Subjekt, umfassend Verabreichen einer wirksamen Einheitsdosisform von 1,4-Dihydro-1-beta-D-ribofuranosyl-3-pyridincarboxamid an das Subjekt mit Bedarf.

2. Zusammensetzung, umfassend 1,4-Dihydro-1-beta-D-ribofuranosyl-3-pyridincarboxamid zur Verwendung bei der Vorbeugung und/oder Behandlung der Nierenerkrankung, die aus akutem Nierenversagen und chronischer Nierenerkrankung ausgewählt ist.

3. Zusammensetzung zur Verwendung nach Anspruch 2, die aus der Gruppe ausgewählt ist, bestehend aus: einem Arzneimittel, einem Lebensmittel- oder Getränkeprodukt, einem Lebensmittelzusatzstoff, einem oralen Nahrungsergänzungsmittel (ONS), einem medizinischen Lebensmittel und Kombinationen davon.

4. Zusammensetzung zur Verwendung nach Anspruch 2 oder 3, wobei das Subjekt ein Mensch ist.

## Revendications

1. 1,4-dihydro-1-bêta-D-ribofuranosyl-3-pyridinecarboxamide pour une utilisation dans la prévention et/ou le traitement des maladies et affections rénales choisies parmi les lésions rénales aiguës et les maladies rénales chroniques, chez un sujet, comprenant l'administration au sujet dans le besoin d'une forme de dose unitaire efficace de 1,4-dihydro-1-bêta-D-ribofuranosyl-3-pyridinecarboxamide.

2. Composition comprenant du 1,4-dihydro-1-bêta-D-ribofuranosyl-3-pyridinecarboxamide pour une utilisation pour prévenir et/ou traiter une maladie rénale choisie parmi une lésion rénale aiguë et une maladie rénale chronique.

3. Composition pour une utilisation selon la revendication 2, qui est choisie dans le groupe constitué de : un produit pharmaceutique, alimentaire ou une boisson, un complément alimentaire, un complément nutritionnel oral (CNO), un aliment médical, et des combinaisons de ceux-ci.

4. Composition pour une utilisation selon la revendication 2 ou 3, dans laquelle le sujet est un être humain.
